# EUROPEAN PATENT APPLICATION

(11) **EP 4 818 734 A1**
(43) Date of publication of application: **07.10.2026**
(21) Application number: 26170080.1
(22) Date of filing: 31.03.2026
(51) Int. Cl.: G01R 33/54

(54) **IMAGING SUPPORT APPARATUS, METHOD OF OPERATING IMAGING SUPPORT APPARATUS, PROGRAM ON A NON-TRANSITORY, COMPUTER READABLE RECORDING MEDIUM FOR CARRYING OUT THE METHOD, AND MAGNETIC RESONANCE IMAGING APPARATUS**

(30) Priority: 01.04.2025 JP 2025060788
(71) Applicant: FUJIFILM Corporation, Tokyo 106-8620 (JP)
(72) Inventor: MORI, Akiko, Tokyo, 1068620 (JP); HATORI, Shohei, Tokyo, 1068620 (JP); SAKURAGI, Kenta, Tokyo, 1068620 (JP); IKEGAWA, Ayaka, Tokyo, 1068620 (JP)
(74) Representative: Dehns Germany Partnerschaft mbB

(57) **Abstract**

Provided are an imaging support apparatus, a method of operating an imaging support apparatus, a program, and a magnetic resonance imaging apparatus that provide a user interface that is easy for a user to use in a case in which a position of a landmark is manually adjusted. An imaging support apparatus acquires three-dimensional data of a heart acquired in scanogram imaging, detects a landmark of the heart from the three-dimensional data, calculates a plurality of cross sections to be applied to imaging of the heart based on a position of the landmark, generates cross-sectional images appearing in the plurality of cross sections, displays the plurality of cross-sectional images, and displays a first movement element representing the position of the landmark in a plane of the respective cross-sectional images and a second movement element representing the position of the landmark in a depth direction of the respective cross-sectional images.

## Description

### BACKGROUND OF THE INVENTION

### 1. Field of the Invention

The present disclosure relates to an imaging support apparatus, a method of operating an imaging support apparatus, a program, and a magnetic resonance imaging apparatus.

### 2. Description of the Related Art

In the cardiac MRI examination, imaging of a reference cross section specified based on anatomical features of a heart is performed. Useful reference cross-sectional images for diagnosis include, for example, a left ventricular short-axis image, a horizontal long-axis image, a vertical long-axis image, a four-chamber long-axis image, a two-chamber long-axis image, a three-chamber long-axis image, and the like, and for each examination, the imaging position of each cross section is set in accordance with an examinee. Note that MRI is an abbreviation for Magnetic Resonance Imaging.

JP2012-110688A discloses a technique of automatically detecting a landmark from three-dimensional data and automatically determining reference six cross sections including the landmark. That is, an MRI apparatus disclosed in JP2012-110688A acquires multi-slice axial cross-sectional image data covering the entire heart of the examinee in preliminary imaging before main imaging, and generates three-dimensional volume image data of the heart using isotropic processing. Further, in the apparatus disclosed in JP2012-110688A, positions of anatomical features of the heart, such as an apex, a mitral valve, a long axis, and a left ventricular center, are detected from three-dimensional volume data, and a cross section is set based on the positions of the anatomical features of the heart. A user such as a radiologist determines an imaging position based on the cross section set based on the anatomical feature of the heart and performs main imaging.

### SUMMARY OF THE INVENTION

For example, there may be a case where the user wants to manually adjust the position of the automatically detected landmark, such as a case where the position of the landmark representing the feature point of the automatically detected anatomical structure of the heart is different from the actual position. However, the apparatus disclosed in JP2012-110688A does not have a function of correcting the position of the automatically detected landmark.

The present disclosure has been made in view of such circumstances, and an object of the present disclosure is to provide an imaging support apparatus, a method of operating an imaging support apparatus, a program, and a magnetic resonance imaging apparatus that provide a user interface that is easy for a user to use in a case where a position of a landmark is manually adjusted.

An imaging support apparatus according to a first aspect of the present disclosure is an imaging support apparatus that supports imaging performed by a magnetic resonance imaging apparatus, the imaging support apparatus including: a processor; and a memory, in which the processor is configured to: acquire three-dimensional data including a heart of an examinee, the three-dimensional data being acquired in scanogram imaging performed using the magnetic resonance imaging apparatus before main imaging; detect a landmark of the heart from the three-dimensional data; calculate, based on a position of the landmark, a plurality of cross sections applied to imaging of the heart; generate a plurality of cross-sectional images appearing in each of the plurality of cross sections from the three-dimensional data; display the plurality of cross-sectional images; display a first movement element representing a position of the landmark in a plane of the respective cross-sectional images; and display a second movement element representing a position of the landmark in a depth direction of the respective cross-sectional images.

In an imaging support apparatus according to a second aspect, according to the imaging support apparatus of the first aspect, the processor may be configured to: receive, for any one cross-sectional image of the plurality of cross-sectional images, an operation of moving the first movement element; and move, in response to the operation of moving the first movement element, the position of the landmark in the plane of the one cross-sectional image.

In an imaging support apparatus according to a third aspect, according to the imaging support apparatus of the second aspect, the processor may be configured to move, in response to the movement of the position of the landmark in the plane of the one cross-sectional image, the position of the landmark in the plane of another cross-sectional image.

In an imaging support apparatus according to a fourth aspect, according to the imaging support apparatus of the third aspect, the processor may be configured to display, for the other cross-sectional image, using the second movement element, a position to which the landmark is moved in the depth direction in a case where the position of the landmark in the depth direction is moved in response to the movement of the position of the landmark in the plane.

In an imaging support apparatus according to a fifth aspect, according to the imaging support apparatus of the fourth aspect, the processor may be configured to change, for the other cross-sectional image, a display mode of the first movement element in a case where the position of the landmark in the depth direction is moved.

In an imaging support apparatus according to a sixth aspect, according to the imaging support apparatus of any one of the first aspect to the fifth aspect, the processor may be configured to: receive, for any one cross-sectional image of the plurality of cross-sectional images, an operation on the second movement element; and move, in response to an operation of moving the position of the landmark in the depth direction represented by the second movement element, the position of the landmark in the depth direction of the one cross-sectional image.

In an imaging support apparatus according to a seventh aspect, according to the imaging support apparatus of the sixth aspect, the processor may be configured to change, for any one cross-sectional image of the plurality of cross-sectional images, a display mode of the first movement element in a case where the position of the landmark in the depth direction is moved.

In an imaging support apparatus according to an eighth aspect, according to the imaging support apparatus of any one of the first aspect to the seventh aspect, the processor may be configured to apply different display modes to the first movement element in accordance with the position of the landmark in the depth direction.

In an imaging support apparatus according to a ninth aspect, according to the imaging support apparatus of any one of the first aspect to the eighth aspect, the processor may be configured to automatically detect the landmark of the heart from the three-dimensional data.

In an imaging support apparatus according to a tenth aspect, according to the imaging support apparatus of any one of the first aspect to the eighth aspect, the processor may be configured to receive a manual setting of the position of the landmark for one or more predefined cross-sectional images.

A method of operating an imaging support apparatus according to an eleventh aspect of the present disclosure is a method of operating an imaging support apparatus, the method including: causing a computer that functions as the imaging support apparatus that supports imaging performed by a magnetic resonance imaging apparatus to execute: a step of acquiring three-dimensional data including a heart of an examinee, the three-dimensional data being acquired in scanogram imaging performed using the magnetic resonance imaging apparatus before main imaging; a step of detecting a landmark of the heart from the three-dimensional data; a step of calculating, based on a position of the landmark, a plurality of cross sections applied to imaging of the heart; a step of generating a plurality of cross-sectional images appearing in each of the plurality of cross sections from the three-dimensional data; a step of displaying the plurality of cross-sectional images; a step of displaying a first movement element representing a position of the landmark in a plane of the respective cross-sectional images; and a step of displaying a second movement element representing a position of the landmark in a depth direction of the respective cross-sectional images.

Configuration requirements of the imaging support apparatus according to the second aspect to the tenth aspect may be applied as configuration requirements of the method of operating an imaging support apparatus according to other aspects.

A program according to a twelfth aspect of the present disclosure is a program including: causing a computer that functions as an imaging support apparatus that supports imaging performed by a magnetic resonance imaging apparatus to implement: a function of acquiring three-dimensional data including a heart of an examinee, the three-dimensional data being acquired in scanogram imaging performed using the magnetic resonance imaging apparatus before main imaging; a function of detecting a landmark of the heart from the three-dimensional data; a function of calculating, based on a position of the landmark, a plurality of cross sections applied to imaging of the heart; a function of generating a plurality of cross-sectional images appearing in each of the plurality of cross sections from the three-dimensional data; a function of displaying the plurality of cross-sectional images; a function of displaying a first movement element representing a position of the landmark in a plane of the respective cross-sectional images; and a function of displaying a second movement element representing a position of the landmark in a depth direction of the respective cross-sectional images.

Configuration requirements of the imaging support apparatus according to the second aspect to the tenth aspect may be applied as configuration requirements of the program according to other aspects.

The present disclosure also includes a non-transitory, tangible computer-readable storage medium having the program according to the twelfth aspect stored therein.

A magnetic resonance imaging apparatus according to a thirteenth aspect of the present disclosure is a magnetic resonance imaging apparatus that generates an image of an examinee based on nuclear magnetic resonance, the magnetic resonance imaging apparatus including: a processor; and a memory, in which the processor is configured to: acquire three-dimensional data including a heart of the examinee, the three-dimensional data being acquired in scanogram imaging performed before main imaging; detect a landmark of the heart from the three-dimensional data; calculate, based on a position of the landmark, a plurality of cross sections applied to imaging of the heart; generate a plurality of cross-sectional images appearing in each of the plurality of cross sections from the three-dimensional data; display the plurality of cross-sectional images; display a first movement element representing a position of the landmark in a plane of the respective cross-sectional images; and display a second movement element representing a position of the landmark in a depth direction of the respective cross-sectional images.

Configuration requirements of the imaging support apparatus according to the second aspect to the tenth aspect may be applied as configuration requirements of the magnetic resonance imaging apparatus according to other aspects.

According to the present disclosure, in a case in which a position of a landmark used for calculating an imaging position in a heart examination is manually adjusted, a user interface that is easy for a user to use is provided.

### BRIEF DESCRIPTION OF THE DRAWINGS

FIG. 1 is a diagram showing a schematic configuration of an MRI apparatus according to a first embodiment.
FIG. 2 is a flowchart showing an example of a procedure of a heart examination applied to the MRI apparatus according to the first embodiment.
FIG. 3 is a diagram showing an example of an automatic setting screen.
FIG. 4 is a flowchart in which a flow of display and processing is added to the procedure of the heart examination shown in FIG. 2.
FIG. 5 is an explanatory diagram of landmark display in an MPR image shown in FIG. 3.
FIG. 6 is a diagram showing an example of an automatic setting screen applied to an MRI apparatus according to a first modification example of the first embodiment.
FIG. 7 is a flowchart showing an example of a procedure of a heart examination applied to the MRI apparatus according to the first modification example.
FIG. 8 is an explanatory diagram of landmark display in an MPR image according to the first modification example.
FIG. 9 is a flowchart showing an example of a procedure of a heart examination applied to an MRI apparatus according to a second modification example of the first embodiment.
FIG. 10 is an explanatory diagram of landmark display in an MPR image according to the second modification example.
FIG. 11 is a flowchart showing a procedure of a heart examination applied to an MRI apparatus according to a second embodiment.
FIG. 12 is a diagram showing an example of a manual setting screen.
FIG. 13 is a diagram showing an example of a transition of the manual setting screen shown in FIG. 12.
FIG. 14 is a flowchart showing an example of a procedure of a heart examination according to a modification example of the second embodiment.
FIG. 15 is a schematic diagram showing an example of a display mode of a marker.
FIG. 16 is a schematic diagram in a case in which a marker is moved in a plane of an MPR image.
FIG. 17 is a schematic diagram in a case in which a marker is moved in a depth direction of an MPR image.
FIG. 18 is a schematic diagram showing an example of a transition of a display mode of a marker.
FIG. 19 is a functional block diagram of an imaging support apparatus.

### DESCRIPTION OF THE PREFERRED EMBODIMENTS

Hereinafter, preferred embodiments of the present invention will be described in detail with reference to the accompanying drawings. In the following description and the accompanying drawings, the same components are denoted by the same reference numerals, and duplicate descriptions will be omitted. In addition, in the following embodiment, in a case in which a plurality of components are described and listed, it can be interpreted that at least one of the plurality of components is included.

### Configuration Example of Magnetic Resonance Imaging Apparatus according to First Embodiment

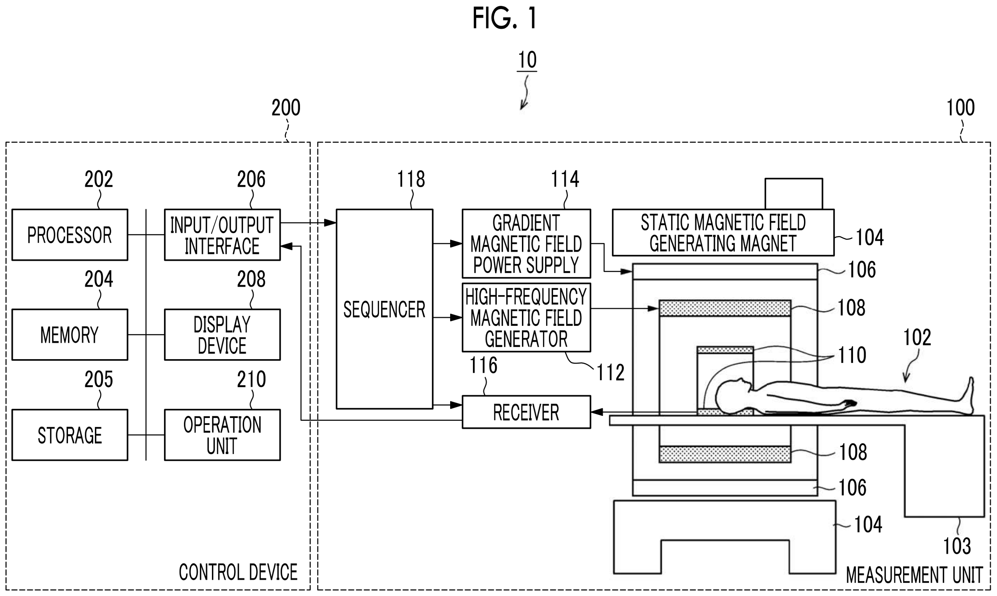

FIG. 1 is a diagram showing a schematic configuration of an MRI apparatus according to a first embodiment. The MRI apparatus may be referred to as a magnetic resonance imaging apparatus. Note that MRI is an abbreviation for Magnetic Resonance Imaging.

An MRI apparatus 10 comprises a measurement unit 100 and a control device 200. The measurement unit 100 comprises a static magnetic field generating magnet 104, a gradient magnetic field coil 106, a radio frequency (RF) coil 108, a high-frequency magnetic field generator 112, a gradient magnetic field power supply 114, a receive coil 110, a receiver 116, and a sequencer 118.

The measurement unit 100 includes a gantry having a cylindrical imaging space called a bore, and elements such as the static magnetic field generating magnet 104, the gradient magnetic field coil 106, and the RF coil 108 are disposed in the gantry. An examinee 102 is usually disposed in the imaging space in the gantry in a state of lying on a bed 103. The bed 103 may be configured to be fixed to the gantry, or may be a movable dockable bed that can be attached to and detached from the gantry.

The static magnetic field generating magnet 104 generates a uniform static magnetic field in the imaging space in which the examinee 102 is disposed. The static magnetic field generating magnet 104 includes a static magnetic field generating source of a permanent magnet type, a normal conducting type, or a superconducting type. The gradient magnetic field coil 106 generates a gradient magnetic field in the imaging space. The RF coil 108 generates a high-frequency magnetic field that causes a nuclear magnetic resonance (NMR) signal to be generated in atomic nuclei of atoms constituting tissues of the examinee 102. The RF coil 108 is also referred to as a transmission coil. The receive coil 110 detects the NMR signal generated from the examinee 102. The receive coil 110 is also referred to as an RF probe.

The sequencer 118 transmits control information to the high-frequency magnetic field generator 112 and the gradient magnetic field power supply 114 in accordance with a pulse sequence (imaging sequence). The high-frequency magnetic field generator 112 generates a high-frequency current pulse of a Larmor frequency that causes nuclear magnetic resonance based on the control information input from the sequencer 118, and transmits the high-frequency current pulse to the RF coil 108. An RF pulse corresponding to the high-frequency current pulse is transmitted from the RF coil 108 to the examinee 102.

The receive coil 110 detects the NMR signal generated by the excitation of the nuclear spins in the examinee 102 by the RF pulse, and transmits the detected NMR signal. The NMR signal is usually collected as a gradient echo or a spin echo, and here, it is referred to as an echo signal. The receive coil 110 shown in FIG. 1 is an example of a coil that receives an echo signal from the head of the examinee 102, but the present invention is not limited to the receive coil that receives the echo signal from the head, and a receive coil corresponding to an examination site such as a chest, an abdomen, a lumbar region, a shoulder, or hands and feet of the examinee 102 is used and appropriately set. In addition, the receive coil also includes a flexible blanket type receive coil that is placed over the chest, the abdomen, and the lumbar region.

The gradient magnetic field coil 106 is composed of an X-axis gradient magnetic field coil that generates a gradient magnetic field Gx in an X-axis direction of the imaging space, a Y-axis gradient magnetic field coil that generates a gradient magnetic field Gy in a Y-axis direction of the imaging space, and a Z-axis gradient magnetic field coil that generates a gradient magnetic field Gz in a Z-axis direction. The gradient magnetic field coil 106 generates a gradient magnetic field corresponding to a current supplied from the gradient magnetic field power supply 114 in the imaging space. In a coordinate system of three orthogonal axes of the imaging space, a direction of a central axis of the gantry is usually defined as a Z-axis direction, a vertical direction is usually defined as a Y-axis direction, and a direction orthogonal to each of the Z-axis and the Y-axis is usually defined as an X-axis direction.

The Z-axis gradient magnetic field coil generates the gradient magnetic field Gz for selecting a slice position and a slice width of a plane of the examinee 102 orthogonal to the Z-axis direction. The X-axis gradient magnetic field coil generates the gradient magnetic field Gx proportional to a position in the X-axis direction as a readout direction gradient magnetic field in a period in which the echo signal is generated. The readout direction may be referred to as a frequency-encoding direction. The Y-axis gradient magnetic field coil generates the gradient magnetic field Gy in the phase-encoding direction having different intensities for each repetition time TR.

The sequencer 118 controls each unit to operate at a timing and intensity programmed in advance. Among the programs, a program describing the timing and the intensity of the RF pulse, the gradient magnetic field Gx, the gradient magnetic field Gy, the gradient magnetic field Gz, and the signal reception is particularly referred to as a pulse sequence.

Various pulse sequences are known depending on the purpose, and examples thereof include a spin echo method and a gradient echo method, and there are various pulse sequences derived from these. In addition, there is echo planar imaging (EPI) which is one of the high-speed imaging methods. The EPI includes single-shot EPI in which a plurality of gradient echoes are created by repeatedly inverting the gradient magnetic field during excitation by one RF pulse and a k-space in a spatial frequency region is filled with data necessary for image reconstruction, and multi-shot EPI in which the k-space is filled with data of an echo train obtained by a plurality of shots.

The NMR signal generated from the examinee 102 is detected by the receive coil 110, is amplified by a preamplifier (not shown) in the receive coil 110, and is transmitted to the receiver 116.

The receiver 116 includes a quadrature detection circuit, an analog to digital (A/D) converter, and other signal processing circuits. In the receiver 116, the amplified NMR signal is subjected to A/D conversion and necessary signal processing to generate data. The data generated in this way is transmitted to the control device 200.

For example, the receiver 116 executes detection and A/D conversion of the echo signal detected by the receive coil 110, and transmits two series of digital data of a real part and an imaginary part to the control device 200. The digital data is also referred to as reception signal data or measurement data.

The sequencer 118 transmits information on a nuclear magnetic resonance frequency (detection reference frequency) that is a reference for quadrature detection performed in the receiver 116, a timing of the A/D conversion (sampling timing in the frequency-encoding direction), and the like, and controls the receiver 116.

A reception-side cable for outputting the NMR signal received by the receive coil 110 is connected to the receive coil 110. A reception-side connector is connected to an end portion of the reception-side cable. The reception-side connector is connected to a bed-side connector provided on the bed 103. The bed-side connector is connected to a bed-side cable disposed inside the bed 103, and the bed-side cable is connected to the control device 200.

As a result, the receive coil 110, the control device 200, and the sequencer 118 are communicably connected to each other. The connection between the receive coil 110, and the control device 200 and the sequencer 118 is not limited to wired connection, such as through a cable, and can also be established wirelessly. In this case, the receive coil 110 or the bed 103 further includes at least an A/D conversion module and a wireless communication module. In addition, the reception-side cable, the reception-side connector, the bed-side connector, and the bed-side cable are not shown.

The control device 200 controls the measurement unit 100 and performs various types of operations such as image reconstruction based on the signal obtained from the measurement unit 100. The control device 200 can be configured by using a computer. The computer applied to the control device 200 may be a personal computer, a workstation, or a server computer.

The control device 200 comprises a processor 202, a memory 204, a storage 205, an input/output interface 206, a display device 208, an operation unit 210, and the like as hardware.

The processor 202 includes a central processing unit (CPU). The processor 202 may include a graphics processing unit (GPU). In addition, the processor 202 may include one or a plurality of pieces of hardware of a digital signal processor (DSP), an application-specific integrated circuit (ASIC), a programmable logic device (PLD), and the like.

The processor 202 performs overall control of each unit of the control device 200 and the measurement unit 100, and executes various programs stored in the memory 204 to realize various functions.

The memory 204 is a storage device including a random access memory (RAM). The memory 204 may include one or more of a flash memory and a read-only memory (ROM). The storage 205 is a storage device including, for example, one or more of a hard disk apparatus, a solid state drive apparatus, and a removable recording medium. The flash memory, the ROM, and the storage 205 are non-volatile storage devices that store an operation system, a program for causing the processor 202 to function as the control device 200, various pulse sequences, calculation expressions used for image reconstruction, parameters, trained models, MR images subjected to image reconstruction, and the like.

The RAM functions as a work area for processing by the processor 202, and temporarily stores programs and the like stored in the non-volatile storage device. In addition, the RAM functions as a place for temporarily storing measurement data, which is digital sampling data obtained by processing the echo signal obtained from the measurement unit 100, and so-called raw data. It should be noted that a part (RAM) of the memory 204 may be built into the processor 202.

The input/output interface 206 includes a communication unit that can be connected to a network, a connection unit that can be connected to an external apparatus, and the like. For example, a universal serial bus (USB), a high-definition multimedia interface (HDMI) (HDMI is a registered trademark), or the like can be applied as the connection unit that can be connected to the external apparatus.

The processor 202 communicates with the sequencer 118 (in the present example) and the receiver 116, which are disposed in the measurement unit 100 via the input/output interface 206, and transmits and receives necessary information. A part of the sequencer 118 may be provided on the control device 200 side.

The display device 208 is configured as, for example, a liquid crystal display, an organic electro-luminescence (OEL) display, a projector, or an appropriate combination thereof. Various types of information are displayed on the display device 208 in addition to the MRI image captured by the MRI apparatus 10. The display device 208 is used as a part of a user interface (UI) in a case of receiving an input from the operation unit 210. The display device 208 is not limited to one, and a multi-display form comprising a plurality of display devices is also possible.

The operation unit 210 includes an input device such as a mouse and a keyboard, and functions as a part of a graphical user interface (GUI) that receives an input from an operator using a display operation window of the display device 208. For example, the operation unit 210 and the display device 208 function as a GUI for an operator to input the start and stop (temporary stop) of the measurement unit 100, the selection of the pulse sequence, the imaging conditions, the processing conditions, and the like. The operation unit 210 may include a voice input device. In addition, the operation unit 210 may be a touch panel type input device configured integrally with a display screen of the display device 208.

A control device 200 shown in FIG. 1 has a function as an imaging support apparatus that supports the capturing of a medical image performed using the MRI apparatus 10. Details of the function of the imaging support apparatus will be described below.

### Procedure of Heart Examination Applied to MRI Apparatus according to First Embodiment

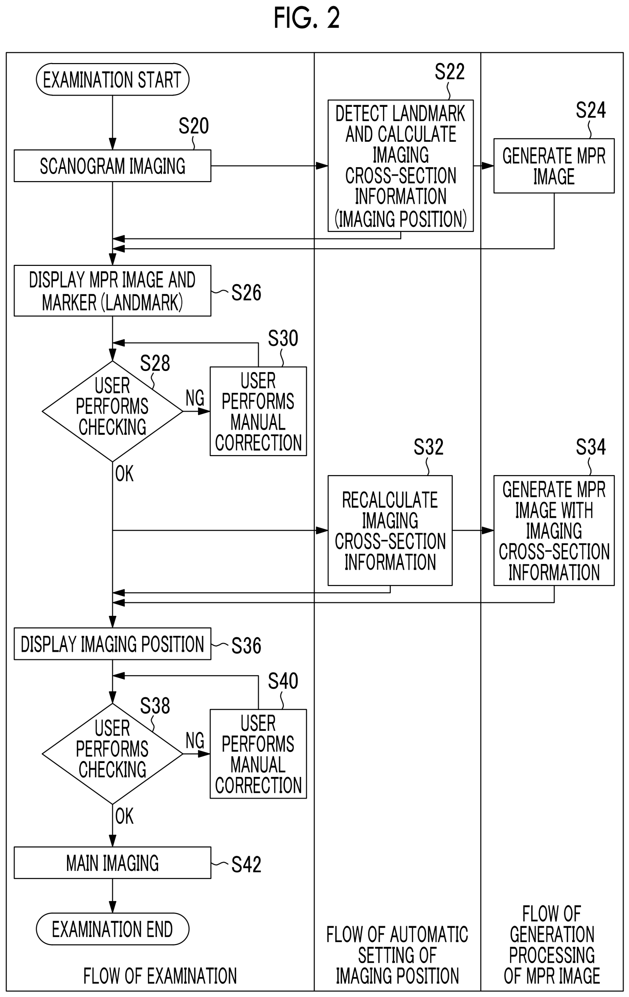

FIG. 2 is a flowchart showing an example of a procedure of a heart examination applied to the MRI apparatus according to the first embodiment. FIG. 2 shows processing of automatically setting an imaging position corresponding to an imaging cross section applied to main imaging in the procedure of the heart examination, and generation of an MPR image of reference six cross sections or the like. The MPR image is any cross-sectional image cut out and reconstructed from three-dimensional data. Note that MPR is an abbreviation for multi-planar reconstruction.

A computer applied to the control device 200 shown in FIG. 1 executes a predetermined program, and a user such as an operator inputs a signal to the control device 200 or the like using an operation unit 210 to execute various types of processing. Note that the procedure of the heart examination shown in FIG. 2 includes a method of operating the imaging support apparatus according to the first embodiment.

In step S20, prior to the main imaging, scanogram (scout) imaging of the heart is performed based on an imaging position set in advance, and three-dimensional data of the heart in the examinee 102 is acquired.

In step S22, a landmark representing a feature point of an anatomical structure of the heart is automatically detected from the three-dimensional data of the heart in the examinee 102 acquired in step S20, and imaging cross-section information including information on an imaging position applied to the main imaging is calculated based on the automatically detected landmark. For example, in step S22, the reference six cross sections are calculated as the imaging cross-section information. Note that the reference six cross sections are examples of a plurality of cross sections of the present disclosure.

For the automatic detection of the landmark, a trained learning model that learns a position of the landmark in two-dimensional data or three-dimensional data of the heart and outputs information representing the position of the landmark of the heart in a case in which the two-dimensional data or the three-dimensional data of the heart is input may be applied.

In addition, for the automatic detection of the feature point (landmark), an algorithm that calculates a position of the feature point based on a shape of the heart acquired from a trained learning model that learns a shape of the heart, such as a contour of a left atrium, in two-dimensional data or three-dimensional data of the heart and outputs information representing the shape of the heart in a case in which the two-dimensional data or the three-dimensional data of the heart is input may be applied.

In step S24, one or more MPR images are generated for each of the plurality of cross sections included in the imaging cross-section information calculated in step S22. For example, in step S24, the MPR image is generated for each of the reference six cross sections. The MPR image in each of the reference six cross sections is an example of a plurality of cross-sectional images of the present disclosure.

In step S26, a marker representing the position of the landmark detected in step S22 is superimposed on the MPR image generated in step S24 and displayed on the display device 208.

In step S28, the user checks the position of the landmark for each MPR image displayed on the display device 208, and in a case in which the position of the landmark is corrected, the process proceeds to step S30. In step S30, the user can operate the operation unit 210 to move the marker and correct the position of the landmark.

In step S28, for example, in a case in which the user operates the operation unit 210 to input a signal for manually moving the marker representing the position of the landmark, the determination result is NG, and the process proceeds to step S30. On the other hand, in a case in which the user operates the operation unit 210 to input a signal representing the adoption of the position of the landmark, the determination result is OK, and the process proceeds to step S36. On the other hand, in a case in which the position of the landmark is corrected and the determination result in step S28 is OK, the process proceeds to step S32.

In step S32, the imaging cross-section information is recalculated based on the corrected position of the landmark. For example, in step S32, the reference six cross sections are recalculated based on the corrected position of the landmark.

In step S34, the MPR image is generated based on the imaging cross-section information recalculated in step S32. For example, in step S34, the MPR image in the reference six cross sections recalculated in step S32 is generated.

In step S36, the imaging cross-section information is displayed on the main imaging plan screen as the imaging position. In step S38, the user checks the imaging position of the main imaging displayed on the display device 208, and in a case in which the imaging position is corrected, the process proceeds to step S40. In step S40, the user can manually correct the imaging position by operating the operation unit 210.

In step S38, for example, in a case in which the user operates the operation unit 210 to input a signal for manually correcting the imaging position, the determination is NG, and the process proceeds to step S40. On the other hand, in a case in which the user operates the operation unit 210 to input a signal representing the imaging position, the determination is OK, and the process proceeds to step S42.

In step S42, the main imaging in which the imaging position determined in step S38 is applied is performed. In the main imaging, the imaging position corresponding to the imaging cross-section information, the imaging conditions such as the imaging method of the imaging cross-section information, and the like are set in the sequencer 118 shown in FIG. 1.

The preliminary imaging may be performed prior to the scanogram imaging shown as step S20 in FIG. 2, and the imaging position in the scanogram imaging may be determined based on the imaging result of the preliminary imaging. In the preliminary imaging, two-dimensional data may be acquired, and an imaging cross-sectional image representing a predetermined cross section of the heart may be generated.

In the present embodiment, an aspect in which three-dimensional data is acquired as the scanogram imaging is exemplified, but two-dimensional data may be acquired by applying an interval of the MPR images and the number of the MPR images to a degree at which the MPR images in each direction necessary for detecting the landmarks can be generated. In the aspect in which the two-dimensional data is acquired, the preliminary imaging may be performed.

In a case in which the scanogram imaging is performed without performing the preliminary imaging, the heart may not be at a preferred position in the three-dimensional data obtained as a result of the scanogram imaging. Therefore, an aspect in which the position of the heart is specified based on the imaging result of the preliminary imaging and the scanogram imaging is performed is preferable.

### Configuration Example of GUI Applied to Automatic Setting

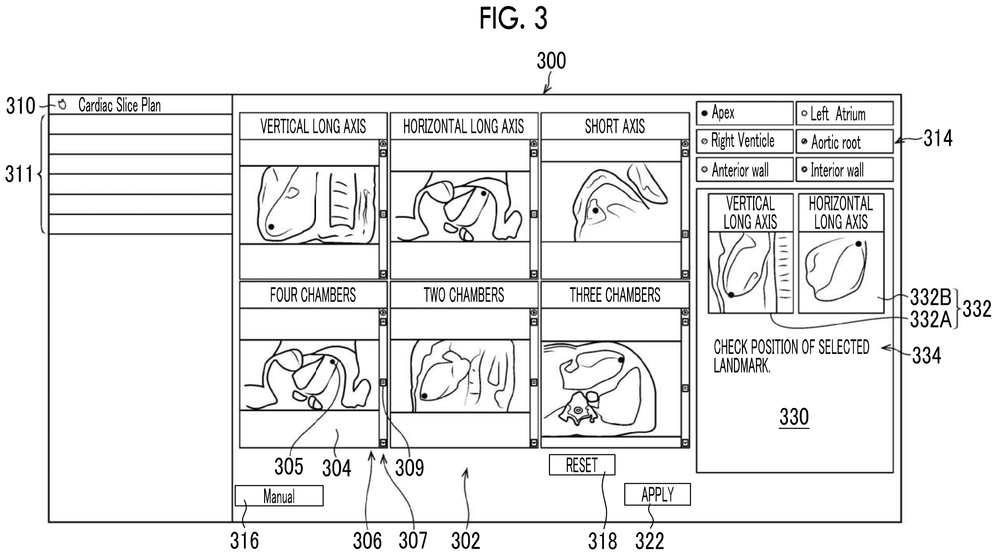

FIG. 3 is a diagram showing an example of an automatic setting screen. An automatic setting screen 300 shown in FIG. 3 is displayed on the display device 208 shown in FIG. 1 in step S26 to step S34 shown in FIG. 2. It should be noted that GUI is an abbreviation for graphical user interface.

The automatic setting screen 300 comprises an MPR image display region 302. A plurality of MPR images 304 corresponding to a plurality of imaging positions are displayed in the MPR image display region 302. An individual window 306 is applied to each MPR image 304 for display.

FIG. 3 shows reference six cross sections as the plurality of imaging positions. That is, FIG. 3 shows the MPR images 304 corresponding to each of a vertical long-axis cross section, a horizontal long-axis cross section, a short-axis cross section, a four-chamber cross section, a two-chamber cross section, and a three-chamber cross section.

A marker 305 indicating a position of a landmark in a plane is superimposed and displayed on the MPR image 304. The user can move the marker 305 by performing drag and drop, for example, by aligning a pointer with the marker 305 in the plane of the MPR image 304.

The window 306 applied to the display of the MPR image 304 comprises a slider bar 307. A position of a slider 309 provided in the slider bar 307 represents a position in a depth direction orthogonal to the cross section represented by the MPR image 304. The term "orthogonal" may include "substantially orthogonal" that can be treated in the same manner as orthogonal even in a case of intersecting at an angle of less than 90 degrees or more than 90 degrees.

The user can move the position of the cross section represented as the MPR image 304 in the depth direction by moving the position of the slider 309. In a case where the position of the cross section in the depth direction is moved, the MPR image 304 is displayed for the cross section at the moved position. The marker 305 is an example of a first movement element of the present disclosure, and the slider bar 307 is an example of a second movement element of the present disclosure.

The automatic setting screen 300 comprises a first setting screen display switching button 310, a second setting screen display switching button 311, a landmark selection button 314, a manual setting button 316, a reset button 318, and an apply button 322.

The first setting screen display switching button 310 is operated in a case of starting the display of the setting screen for examination. FIG. 3 illustrates the first setting screen display switching button 310 for starting the display of the setting screen for heart examination.

The second setting screen display switching button 311 is operated in a case of starting the display of the imaging plan screen. FIG. 3 illustrates the second setting screen display switching button 311 including six buttons corresponding to the six reference cross sections. The imaging plan screen is not shown.

The landmark selection button 314 is operated in a case of selecting the landmark to be displayed in a superimposed manner on the MPR image 304. FIG. 3 illustrates six landmark selection buttons 314 corresponding to the apex, the center of the left atrium, the sharp edge portion of the right ventricle, the aortic root, the anterior wall center, and the inferior wall center.

The manual setting button 316 is operated in a case of performing the manual setting of the landmark. In a case in which the user operates the manual setting button 316, switching from the automatic setting screen 300 to the manual setting screen applied to the manual setting of the landmark is performed. The reset button 318 is operated in a case of returning the position of the landmark manually moved to the automatically detected position.

The apply button 322 is operated in a case of confirming the position of the landmark represented as the position of the marker 305 of the MPR image 304 displayed on the automatic setting screen 300 and confirming the imaging cross-section information (imaging position) determined from the position of the landmark. Various buttons such as the first setting screen display switching button 310 function as the operation unit 210 shown in FIG. 1.

The automatic setting screen 300 comprises a landmark position sample region 330. In the landmark position sample region 330, a landmark position sample image 332 representing a sample of the position of the landmark is displayed.

FIG. 3 shows a landmark position sample image 332A representing a position of a cardiac apex in a vertical long-axis cross section and a landmark position sample image 332B representing a position of the cardiac apex in a horizontal long-axis cross section, in a case in which the cardiac apex is selected as the landmark. The landmark position sample image 332 may be that of any examinee and may be prepared and stored in advance.

Character information 334 related to automatic detection of the landmark may be displayed in the landmark position sample region 330. FIG. 3 shows character information prompting checking of the position of the automatically detected landmark as the character information 334 related to automatic detection of the landmark.

FIG. 3 shows an aspect in which the MPR image 304 is generated in the six reference cross sections, but the type of the cross section and the number of the cross sections are appropriately defined according to the position of interest in the heart examination.

### Example of Display Control in Flow of Heart Examination

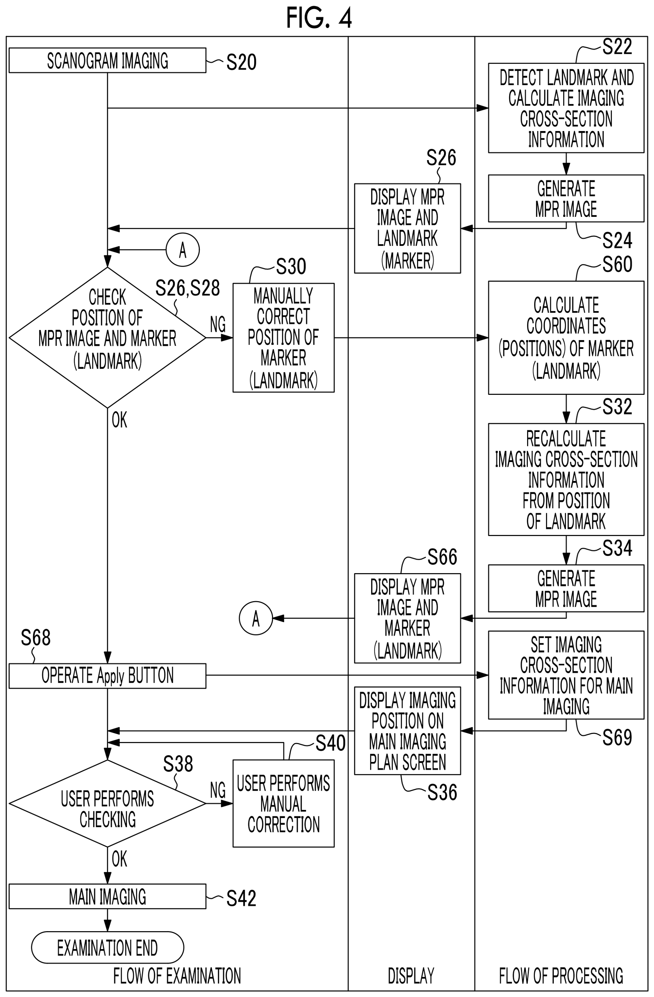

FIG. 4 is a flowchart in which a flow of display and processing is added to the procedure of the heart examination shown in FIG. 2. In a case in which the user operates a first setting screen display switching button 310 on the automatic setting screen 300 shown in FIG. 3, the display of the setting screen for examination is started, and a program for executing a series of procedures related to the examination is activated. FIG. 4 shows an example of the examination of the heart.

In response to the start of the series of procedures related to the examination, the program for implementing the imaging position automatic setting function is activated, and the three-dimensional data is acquired in step S20 of FIG. 4.

The MPR image 304 generated in step S24 is displayed in the MPR image display region 302. In a case in which a button corresponding to a desired landmark is operated from the landmark selection buttons 314 and the desired landmark is selected, a landmark position sample image 332 is displayed in the landmark position sample region 330. In addition, in step S26, a marker 305 representing the position of the landmark is superimposed on the MPR image 304 and displayed.

In a case in which a button corresponding to another landmark different from the displayed landmark is operated from the landmark selection buttons 314, the landmark displayed on the MPR image 304 is changed to a newly selected landmark, and the landmark position sample image 332 is changed to the landmark position sample image 332 corresponding to the newly selected landmark.

In step S30, in a case in which the position of the marker 305 of any MPR image 304 is corrected, the process proceeds to step S60. In step S60, the coordinates (position) of the landmark are automatically calculated from the corrected position of the marker 305, and the process proceeds to step S32. In step S32, the imaging cross-section information is automatically recalculated from the landmark position represented by the marker 305, and the process proceeds to step S34. It should be noted that any MPR image 304 is an example of one cross-sectional image according to the present disclosure.

In step S34, the MPR image 304 for each cross section is generated again based on the recalculated imaging cross-section information, and the process proceeds to step S66. In step S66, the display of the MPR image 304 and the marker 305 is automatically updated.

The process proceeds from step S66 to step S28, and each step from step S28 to step S68 is repeatedly executed until an OK determination is made in step S28. In a case in which an OK determination is made in step S28, the process proceeds to step S68.

In a case in which the apply button 322 is operated in step S68, the position of the landmark represented by the marker 305 is confirmed, the imaging cross-section information (imaging position) determined from the position of the landmark is confirmed, and the process proceeds to step S69.

In step S69, the imaging cross-section information is set for the main imaging, and the process proceeds to step S36. In step S36, the imaging position is displayed on the main imaging plan screen, and the process proceeds to step S38.

In step S36, for example, three cross sections that include at least one landmark and are orthogonal to each other are set based on the confirmed position of the landmark, and at least one of the information on the center position, the angle, or the range of the main imaging region is set for the three cross sections.

In step S38, the user checks the imaging position applied to the main imaging, and in step S40, the imaging position is appropriately corrected. In a case in which the imaging position is confirmed, the main imaging is performed in step S42.

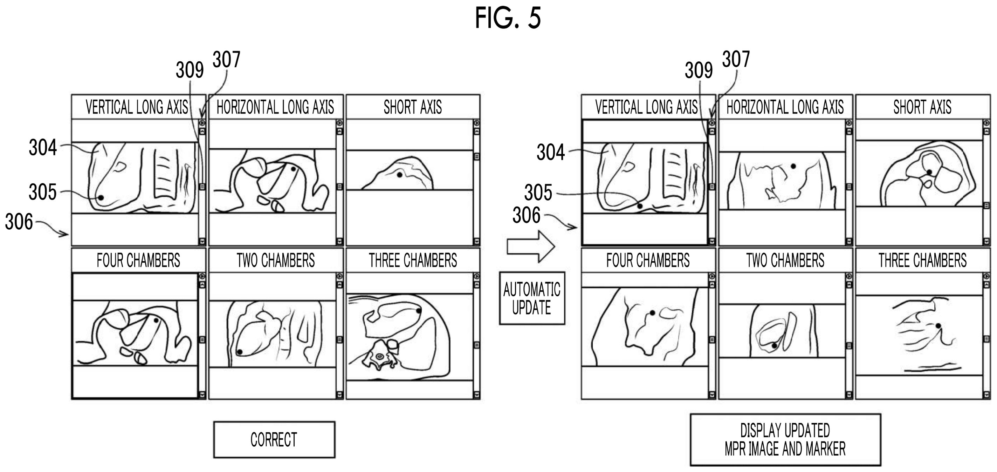

FIG. 5 is an explanatory diagram of landmark display in the MPR image shown in FIG. 3. FIG. 5 shows an MPR image 304 corresponding to each of the vertical long-axis cross section and the like shown in FIG. 3.

The left diagram of FIG. 5 shows the MPR image 304 and the marker 305 before the marker 305 is corrected. The right diagram of FIG. 5 shows the updated MPR image 304 and the marker 305.

For example, in a case in which the user moves the marker 305 in the MPR image 304 of the vertical long-axis cross section from the position in the left diagram to the position in the right diagram, the position of the landmark is calculated based on the position of the moved marker 305, and the imaging cross-section information is calculated based on the position of the landmark. The movement of the marker 305 referred to here may be the correction of the marker 305.

The MPR image 304 is generated for each cross section based on the imaging cross-section information, and the display of the marker 305 is automatically updated for each MPR image 304.

In a case in which the movement of the marker 305 in the MPR image 304 of the vertical long-axis cross section is accompanied by movement in the depth direction in another MPR image 304, the position in the depth direction represented by the MPR image 304 is changed, and the MPR image 304 at the changed position in the depth direction is displayed.

In the example shown in FIG. 5, the position in the depth direction and the position in the horizontal direction are changed for the horizontal long-axis cross section, the short-axis cross section, the four-chamber cross section, the two-chamber cross section, and the three-chamber cross section, and the MPR image 304 at the changed position in the depth direction and the position in the horizontal direction is displayed.

### Effect of First Embodiment

In the first embodiment, the following actions and effects can be obtained.
[1]
   The landmarks are automatically detected from the three-dimensional data of the heart acquired by performing the scanogram imaging in the heart examination of the examinee 102. The MPR image 304 is generated for each of the reference six cross sections based on the automatically detected landmarks. The marker 305 representing the position of the landmark is superimposed and displayed on each of the MPR images 304 for each cross section. The marker 305 in each MPR image 304 can be moved in the plane for each MPR image 304.
   As a result, the user can manually correct the position of the landmark by moving the marker 305 in the plane for each MPR image 304.
[2]
   The window 306 in which the MPR image 304 is displayed comprises the slider bar 307 including the slider 309 representing the position in the depth direction orthogonal to the cross section in the cross section represented as the MPR image 304. As a result, the position of the displayed MPR image 304 in the depth direction is understood based on the position of the slider 309. In addition, by operating the slider 309, the MPR image 304 having a different position in the depth direction is displayed for the displayed MPR image 304.
[3]
   In a case where the position of the marker 305 is moved in any one MPR image 304, the position of the marker 305 in the other MPR images is automatically updated and displayed. As a result, it is possible to understand the position of the landmark corrected in any one of the six reference cross sections in all of the six reference cross sections.

### First Modification Example of First Embodiment

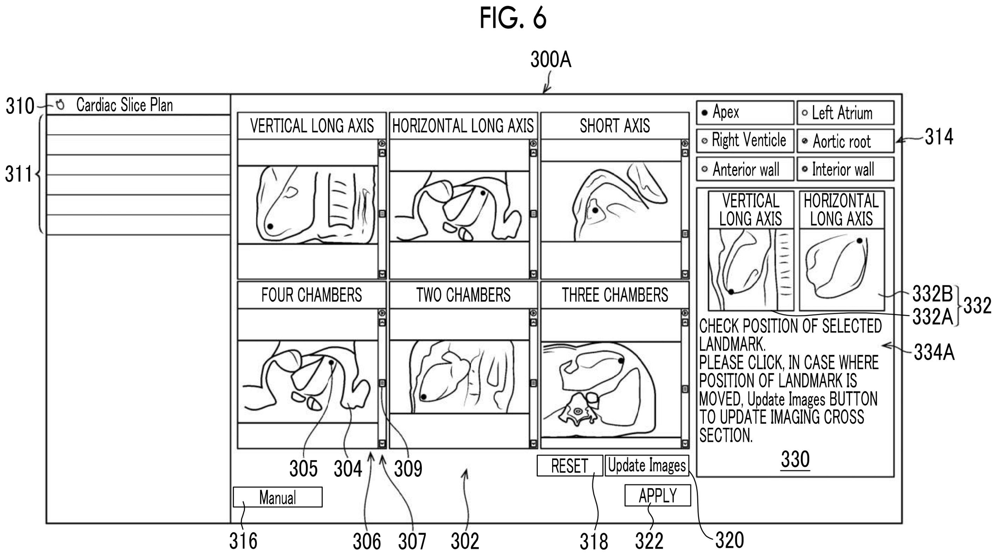

FIG. 6 is a diagram showing an example of an automatic setting screen applied to the MRI apparatus according to the first modification example of the first embodiment. In the first modification example, even in a case where the marker 305 in any MPR image 304 is moved, the MPR image 304 is not automatically updated. In addition, in a case where the manual update of the MPR image 304 is received at any timing, the update of the display of the marker 305 is performed.

An automatic setting screen 300A shown in FIG. 6 is obtained by adding an update image button 320 to the automatic setting screen 300 shown in FIG. 3. The update image button 320 is operated in a case where the MPR image is updated based on the position of the landmark represented as the position of the corrected marker 305.

As shown in FIG. 6, character information 334A in the automatic setting screen 300A may include character information related to the processing in a case where the position of the landmark is moved.

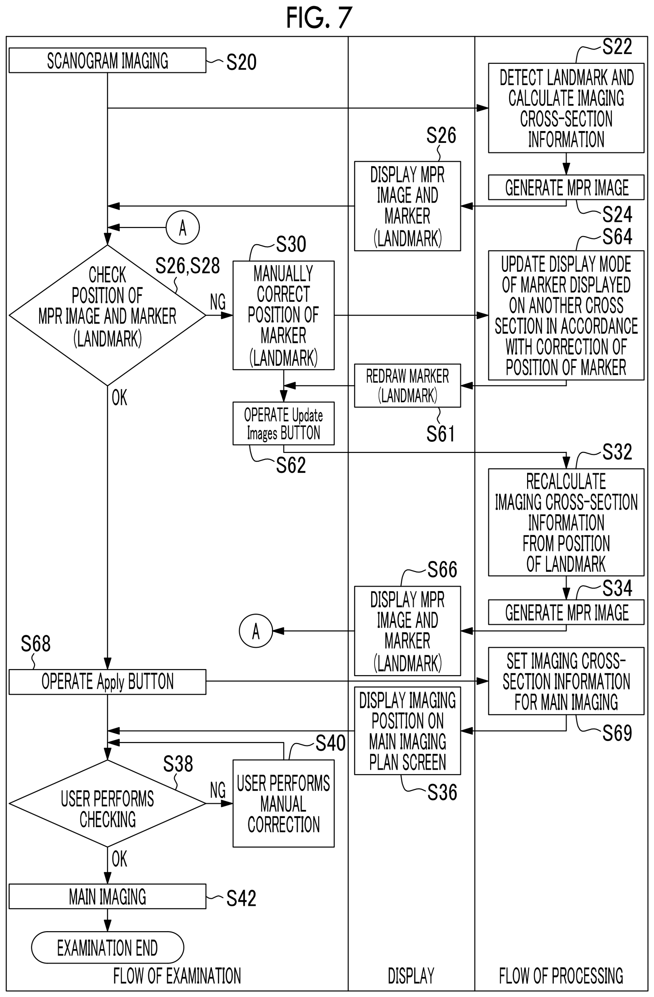

FIG. 7 is a flowchart showing an example of a procedure of a heart examination applied to the first modification example. The procedure of the heart examination shown in FIG. 7 is obtained by adding step S61 to the procedure of the heart examination shown in FIG. 4 and including step S64 instead of step S60 in FIG. 4.

In a case where the position of the marker 305 is manually corrected in step S30, the processing proceeds to step S64. In step S64, the position and the display mode of the marker displayed in the other cross section are changed in accordance with the manually corrected position of the marker 305, and the processing proceeds to step S61. Details of the change in the display mode of the marker 305 will be described below.

In step S61, the marker 305 that is manually corrected and the marker 305 in the other cross section are redrawn (redisplayed), and the processing proceeds to step S62. In step S62, the operation of the update image button 320 shown in FIG. 6 is received. In step S62, in a case where the operation of the update image button 320 is received, the processing proceeds to step S32.

In step S32, the imaging cross-section information is recalculated based on the manually corrected position of the marker 305, and the processing proceeds to step S34. In step S34, the MPR image is generated based on the recalculated imaging cross-section information, and the processing proceeds to step S66.

In step S66, the MPR image 304 and the marker 305 generated in step S34 are displayed, and the processing proceeds to step S28. In step S28, each step from step S30 to step S66 is repeatedly executed until the OK determination is made.

In step S68, in a case where the user operates the apply button 322 and the operation of the apply button 322 is received, the position of the landmark and the imaging cross-section information determined from the landmark position are confirmed, and the processing proceeds to step S69, and each step of step S69, step S36, step S38, step S40, and step S42 is executed in order.

In addition, in step S36, in a case where the second setting screen display switching button 311 shown in FIG. 3 is operated and the operation on the second setting screen display switching button 311 is received, the imaging plan screen is displayed.

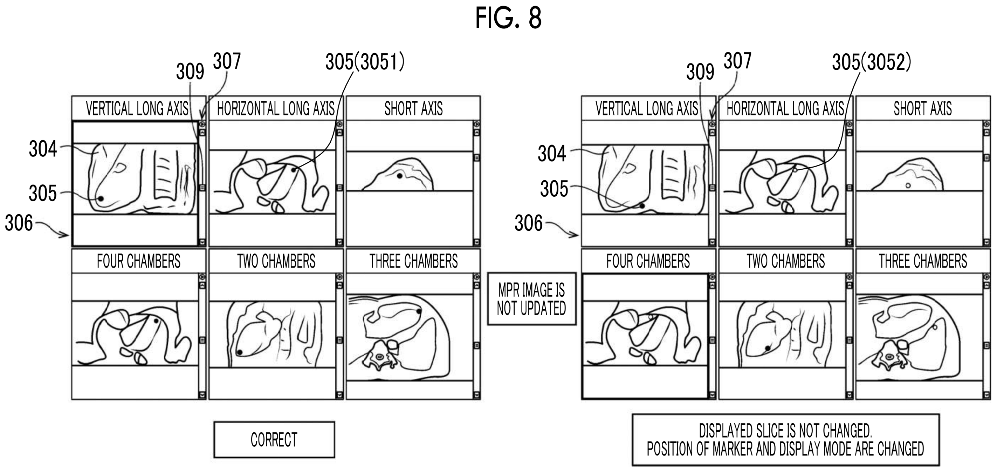

FIG. 8 is an explanatory diagram of the landmark display in the MPR image according to the first modification example. In FIG. 8, as in FIG. 5, the MPR image 304 corresponding to each of the vertical long-axis cross section and the like shown in FIG. 3 is shown.

In the left diagram of FIG. 8, as in the left diagram of FIG. 5, the MPR image 304 and the marker 305 before the marker 305 is moved are shown. In the right diagram of FIG. 8, each MPR image 304 in which the position of the marker 305 is moved, which is displayed in step S61 of FIG. 7, is shown.

In a case where the marker 305 is moved in any MPR image 304, the position of the marker 305 for each MPR image 304 is updated, and the display mode of the other MPR image 304 of the MPR image 304 in which the marker 305 is moved is changed according to the position of the landmark in the depth direction. On the other hand, the MPR image 304 is not updated.

That is, in a case where the position of the landmark in the depth direction is different from the position of the displayed MPR image 304 in the depth direction, the display mode of the marker 305 is changed from a first display mode 3051 to a second display mode 3052.

For example, the marker 305 of the MPR image 304 of the horizontal long-axis cross section is changed from the first display mode 3051 to the second display mode 3052. FIG. 8 shows an aspect in which a solid circle is applied as the first display mode of the marker 305 and a hollow circle is applied as the second display mode. The solid circle represents a circle in which the inside is filled. The hollow circle represents a circle in which the inside is not filled.

### Second Modification Example of First Embodiment

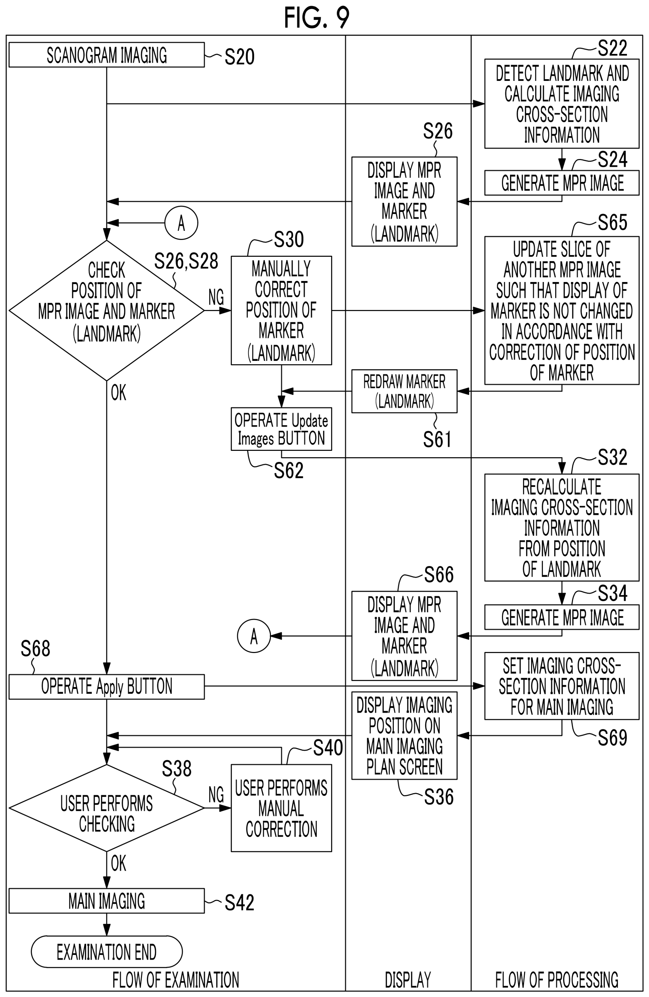

FIG. 9 is a flowchart showing an example of a procedure of a heart examination applied to the MRI apparatus according to the second modification example of the first embodiment. In the second modification example, in a case where the marker 305 is moved in any MPR image 304, the slice position is automatically sent such that the display mode of the marker 305 is not changed in the other MPR images 304.

The procedure shown in FIG. 9 includes step S65 instead of step S64 of the procedure shown in FIG. 7. In step S65, the position of the MPR image 304 in the depth direction in the other cross section is changed such that the first display mode 3051 of the marker 305 is maintained in accordance with the position where the marker 305 is moved.

That is, in step S65, the position in the depth direction in which the recalculated landmark is present is calculated for each cross section represented by the MPR image 304 based on the position of the moved marker 305.

In step S61, the MPR image 304 at the changed position in the depth direction and the marker 305 of which the position is updated are displayed. Each step from step S62 to step S42 is the same as the procedure shown in FIG. 7.

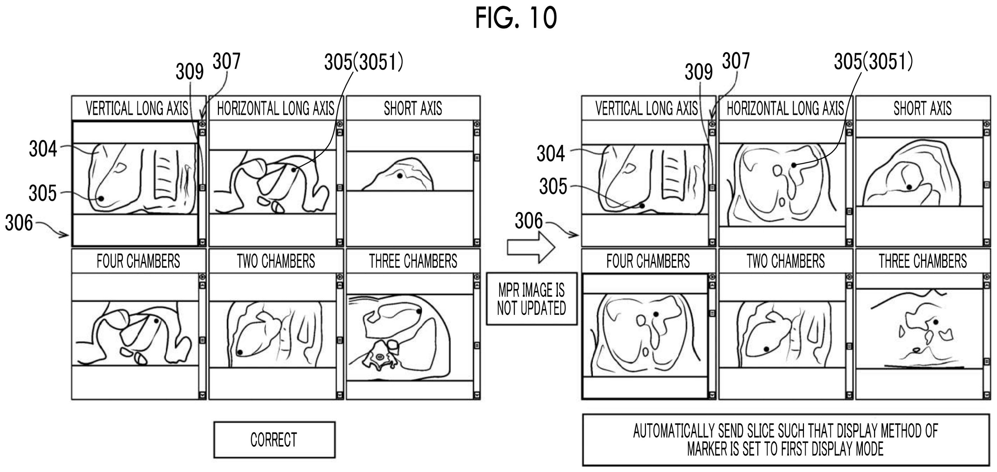

FIG. 10 is an explanatory diagram of landmark display in the MPR image according to the second modification example. In FIG. 8, as in FIG. 5, the MPR image 304 corresponding to each of the vertical long-axis cross section and the like shown in FIG. 3 is shown.

In the left diagram of FIG. 10, the MPR image 304 and the marker 305 before the marker 305 is moved are shown in the same manner as in the left diagram of FIG. 5. In the right diagram of FIG. 10, each MPR image 304 of which the position of the marker 305 is moved, which is displayed in step S61 of FIG. 9, is shown in the same manner as in the right diagram of FIG. 8.

In a case in which the marker 305 is moved in any MPR image 304, the position of the marker 305 for each MPR image 304 is updated, and the MPR image 304 corresponding to the position in the depth direction for each cross section is displayed such that the first display mode 3051 of the marker 305 is maintained.

For example, the position of the marker 305 of the MPR image 304 of the horizontal long-axis cross section is updated in accordance with the movement of the marker 305 of the vertical long-axis cross section. On the other hand, the first display mode 3051 of the marker 305 of the MPR image 304 of the horizontal long-axis cross section is maintained, and the slice position of the MPR image 304 of the horizontal long-axis cross section is changed.

The MRI apparatus 10 may be configured to freely switch between the aspect shown in FIG. 5, the aspect shown in FIG. 8, and the aspect shown in FIG. 10 for the display of the MPR image 304 and the marker 305.

### Effects of Modification Example of First Embodiment

The modification example of the first embodiment can obtain the following effects.
[1]
   In a case in which the position of the marker 305 is moved in any MPR image 304, the position of the landmark is recalculated based on the position of the marker 305 after the movement, and the position of the marker for each MPR image is updated based on the updated position of the landmark. As a result, it is possible to visually recognize the position of the landmark for the MPR image 304 other than the MPR image 304 in which the marker 305 is moved.
[2]
   In a case in which the updated position of the landmark is accompanied by a movement of the position in the depth direction for the MPR image 304 other than the MPR image 304 in which the marker 305 is moved, the display mode of the marker 305 is changed without changing the MPR image 304. As a result, the movement of the position of the landmark in the depth direction is understood in the other MPR image 304.
[3]
   For the MPR image 304 other than the MPR image 304 in which the marker 305 is moved, the MPR image 304 is changed to the MPR image corresponding to the position of the landmark in the depth direction such that the display mode of the marker 305 is not changed in accordance with the update of the position of the landmark. As a result, the MPR image at the position of the landmark in the depth direction and the position in the depth direction in which the landmark is present is understood in each cross section.

### Example of Procedure of Heart Examination Applied to MRI Apparatus according to Second Embodiment

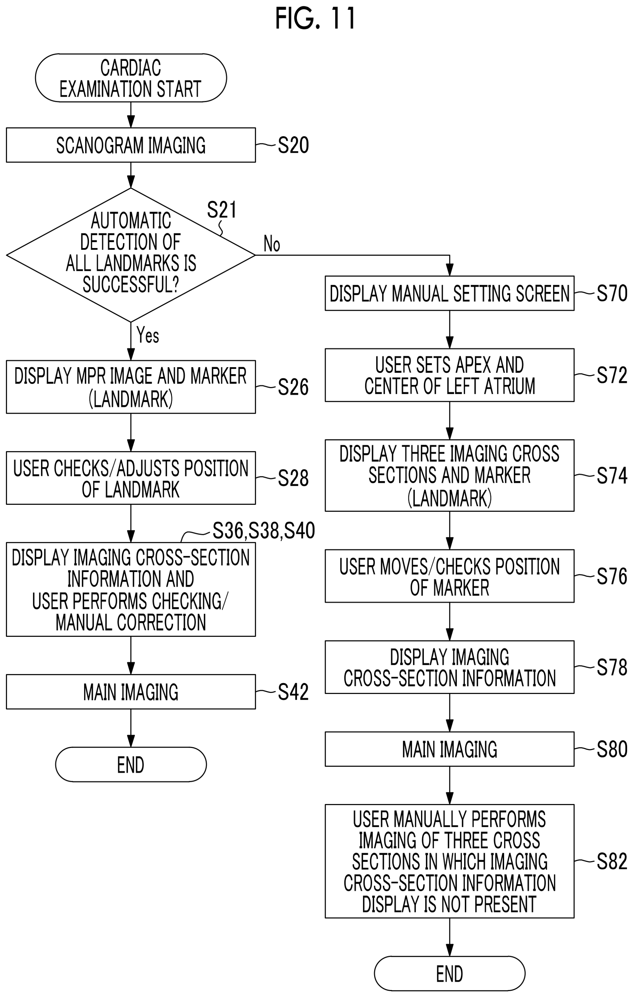

FIG. 11 is a flowchart showing a procedure of a heart examination applied to the MRI apparatus according to the second embodiment. Hereinafter, the differences from the first embodiment will be mainly described, and the description of the same points as those in the first embodiment will be omitted as appropriate.

In the imaging support method according to the second embodiment, the landmark is manually set from the three-dimensional data acquired by performing the scanogram imaging. That is, in step S21, it is determined whether or not the automatic detection of all the landmarks is successful from the three-dimensional data acquired by performing step S20.

In step S21, in a case where it is determined that the automatic detection of all the landmarks is successful, a Yes determination is made, and the procedure from step S22 to step S42 shown in FIG. 2 is performed. In FIG. 11, the illustration of step S22, step S24, step S30, step S32, and step S34 is omitted.

On the other hand, in step S21 shown in FIG. 11, in a case where it is determined that the automatic detection of at least a part of the landmarks has failed, a No determination is made. In a case where a No determination is made, the procedure proceeds to step S70.

In step S21, it is determined whether or not the manual setting button 316 shown in FIG. 3 is operated, and the process may proceed to step S70 in a case where the manual setting button 316 is operated, and may proceed to step S26 in a case where the manual setting button 316 is not operated.

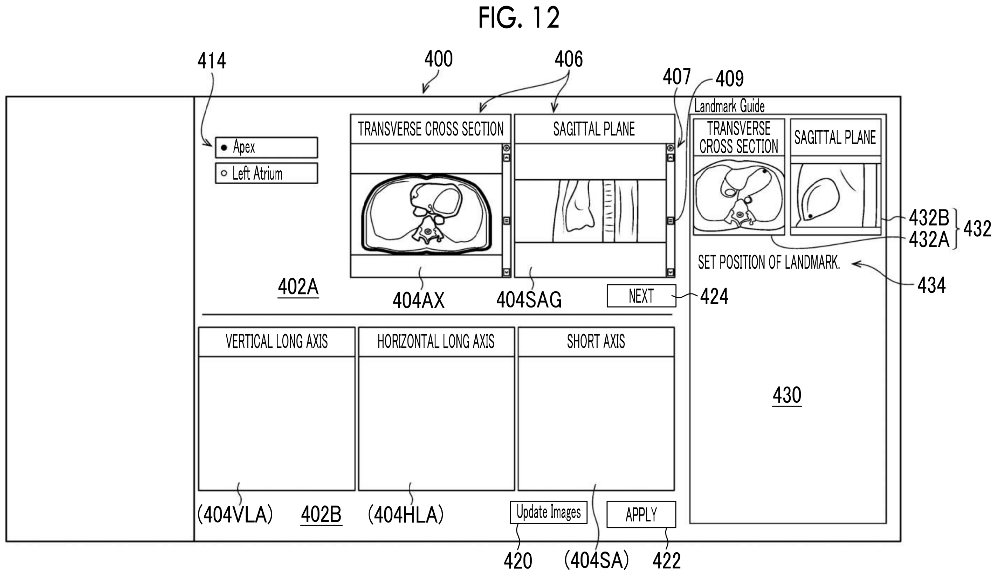

In step S70, a manual setting screen is displayed on the display device 208 shown in FIG. 1 instead of the automatic setting screen 300 shown in FIG. 3. The manual setting screen is denoted by reference numeral 400 and is shown in FIG. 12. In step S72, the user manually sets the apex and the center of the left atrium as the landmarks on the transverse cross-sectional image and the sagittal plane image displayed on the manual setting screen. As a result, the landmarks required for generating the imaging cross-section information are set for the three-dimensional data acquired by performing the scanogram imaging.

The manual setting of the landmarks performed in step S72 is an example of the detection of the landmarks according to the present disclosure. In addition, at least one of the transverse cross-sectional image or the sagittal plane image is an example of one or more cross-sectional images defined in advance according to the present disclosure.

In step S72, the user performs an operation of inputting a signal indicating that the manual setting of the landmarks is ended. The control device 200 receives the signal indicating that the manual setting of the landmarks is ended.

In step S74, the imaging cross-section information and the MPR image are generated for each of the vertical long-axis cross section, the horizontal long-axis cross section, and the short-axis cross section, which are the three imaging cross sections, based on the landmarks set in step S72, and the marker representing the position of the landmark is superimposed and displayed on each MPR image.

In step S76, the user checks the position of the landmark from the position of the marker displayed on the manual setting screen, and manually moves the position of the marker in a case where the position of the landmark needs to be corrected. The control device 200 shown in FIG. 1 receives a signal representing the movement of the position of the marker. Step S76 corresponds to step S28 and step S30 shown in FIG. 2.

In step S78, the imaging cross-section information is recalculated based on the position of the landmark corrected in step S76, and the MPR image based on the recalculated imaging cross-section information is generated. The recalculation of the imaging cross-section information and the generation of the MPR image correspond to step S32 and step S34 in FIG. 2. In addition, in step S78, the MPR image and the imaging cross-section information (imaging position) are displayed on the display device 208. The display of the MPR image corresponds to step S36.

In step S80, the main imaging in which the imaging position based on the imaging cross-section information calculated in step S76 is applied is performed. In step S82, the user manually sets each of the four-chamber cross section, the two-chamber cross section, and the three-chamber cross section, which are applied to the main imaging but are not displayed on the manual setting screen.

In this way, even in a case in which the automatic detection of the landmark is not successful, the imaging position in the main imaging is calculated based on the manually set landmark, and the main imaging is performed.

### Configuration Example of GUI Applied to Manual Setting

FIG. 12 is a diagram showing an example of a manual setting screen. In a case in which the automatic detection of the landmark fails in step S21 of FIG. 11 and in a case in which the manual setting button 316 on the automatic setting screen 300 shown in FIG. 3 is operated, a manual setting screen 400 is displayed on the display device 208 shown in FIG. 1.

The manual setting screen 400 comprises a first MPR image display region 402A and a second MPR image display region 402B. In the first MPR image display region 402A, a landmark selection button 414, a transverse cross-sectional image 404AX, a sagittal plane image 404SAG, and a next button 424 are displayed.

An individual window 406 is applied to each of the transverse cross-sectional image 404AX and the sagittal plane image 404SAG. Each window 406 comprises a slider bar 407 comprising a slider 409 representing a position in the depth direction, such as a cross section.

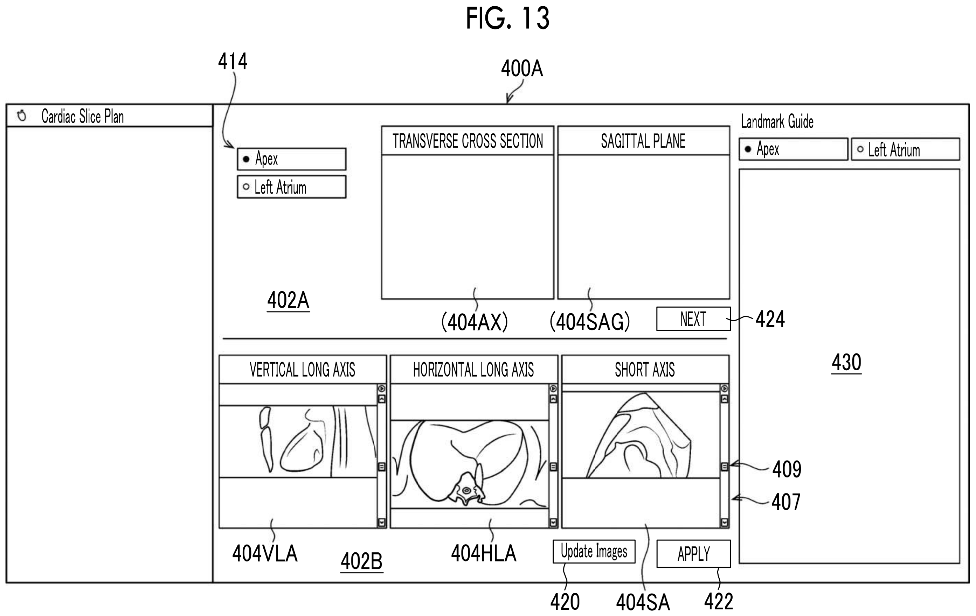

The next button 424 is operated in a case in which the position of the manually set landmark is confirmed. In a case in which the next button 424 is operated, the manual setting screen 400 transitions to a manual setting screen 400A shown in FIG. 13.

In the second MPR image display region 402B shown in FIG. 12, a window of a vertical long-axis cross-sectional image 404VLA, a horizontal long-axis cross-sectional image 404HLA, a short-axis cross-sectional image 404SA, an update image button 420, and an apply button 422 is displayed. FIG. 12 shows the second MPR image display region 402B in a state in which the vertical long-axis cross-sectional image 404VLA, the horizontal long-axis cross-sectional image 404HLA, and the short-axis cross-sectional image 404SA are not displayed.

The vertical long-axis cross-sectional image 404VLA, the horizontal long-axis cross-sectional image 404HLA, and the short-axis cross-sectional image 404SA are examples of a plurality of cross-sectional images appearing in each of a plurality of cross sections of the present disclosure, and the vertical long-axis cross section, the horizontal long-axis cross section, and the short-axis cross section are examples of the plurality of cross sections of the present disclosure.

The update image button 420 is operated in a case in which the position of the marker is moved in any of the vertical long-axis cross-sectional image 404VLA, the horizontal long-axis cross-sectional image 404HLA, or the short-axis cross-sectional image 404SA and the MPR image is updated based on the position of the landmark represented by the moved position of the marker, similarly to the update image button 320 shown in FIG. 3.

The apply button 422 is operated in a case in which the position of the landmark represented by the position of the marker is confirmed and the imaging cross-section information (imaging position) determined from the position of the landmark is confirmed, similarly to the apply button 322 shown in FIG. 3.

The manual setting screen 400 comprises a landmark position sample region 430. In the landmark position sample region 430, a landmark position sample image 432 representing a sample of the position of the landmark is displayed. FIG. 12 shows, as the landmark position sample image 432, a transverse cross-sectional sample position image 432A representing a sample of the position of the landmark in the transverse cross-sectional image 404AX and a sagittal plane sample position image 432B representing a sample of the position of the landmark in the sagittal plane image 404SAG. In the landmark position sample region 430, character information 434 related to the manual setting of the landmark may be displayed.

FIG. 13 is a diagram showing an example of a transition of the manual setting screen shown in FIG. 12. In a manual setting screen 400A shown in FIG. 13, the transverse cross-sectional image 404AX and the sagittal plane image 404SAG shown in FIG. 12 are not displayed, and the transverse cross-sectional sample position image 432A and the like of the landmark position sample region 430 are not displayed.

On the other hand, in the manual setting screen 400A, a vertical long-axis cross-sectional image 404VLA, a horizontal long-axis cross-sectional image 404HLA, and a short-axis cross-sectional image 404SA generated based on the position of the landmark in the transverse cross-sectional image 404AX and the sagittal plane image 404SAG are displayed.

In each of the vertical long-axis cross-sectional image 404VLA, the horizontal long-axis cross-sectional image 404HLA, and the short-axis cross-sectional image 404SA, a marker representing the position of the landmark selected using the landmark selection button 414 is displayed. In the manual setting screen 400A shown in FIG. 13, a marker representing the position of the cardiac apex is displayed as the position of the landmark. In FIG. 13, the marker is not shown.

The update image button 420 has the same function as the update image button 420 shown in FIG. 12.

The apply button 422 has the same function as the apply button 422 shown in FIG. 12.

### Operation and Effect of Second Embodiment

In the second embodiment, the following effects can be obtained.
[1]
   In a case where the automatic setting of the landmark fails, the manual setting screen 400 shown in FIG. 12 is displayed. As a result, the user can manually set the landmark.
[2]
   In the manual setting screen 400, the transverse cross-sectional image 404AX and the sagittal plane image 404SAG are displayed, and a button for selecting the apex or a button for selecting the center of the left atrium is displayed as the landmark selection button 414. As a result, the user can set the apex or the center of the left atrium as the landmark for the transverse cross-sectional image 404AX and the sagittal plane image 404SAG.
[3]
   In a case where the next button 424 is operated on the manual setting screen 400, the vertical long-axis cross-sectional image 404VLA, the horizontal long-axis cross-sectional image 404HLA, and the short-axis cross-sectional image 404SA are generated and displayed based on the landmark set in the transverse cross-sectional image 404AX and the sagittal plane image 404SAG. In the vertical long-axis cross-sectional image 404VLA and the like, a marker indicating the position of the landmark is displayed. As a result, the user can check the position of the landmark in the vertical long-axis cross-sectional image 404VLA and the like. In addition, the user can correct the position of the landmark in the vertical long-axis cross-sectional image 404VLA and the like by moving the marker.
[4]
   In a case where the user moves the position of the marker and operates the update image button 420 in the vertical long-axis cross-sectional image 404VLA and the like, the vertical long-axis cross-sectional image 404VLA and the like are generated again based on the position of the landmark corresponding to the moved position of the marker, and are displayed. As a result, the user can check the position of the landmark corresponding to the moved position of the marker, and can check the vertical long-axis cross-sectional image 404VLA and the like based on the position of the landmark.
[5]
   In a case where the apply button 422 is operated, the position of the landmark is confirmed, and the imaging cross-section information (imaging position) determined from the position of the landmark is confirmed. As a result, it is possible to perform the main imaging in which the imaging position based on the manually set landmark is applied.

### Modification Example of Second Embodiment

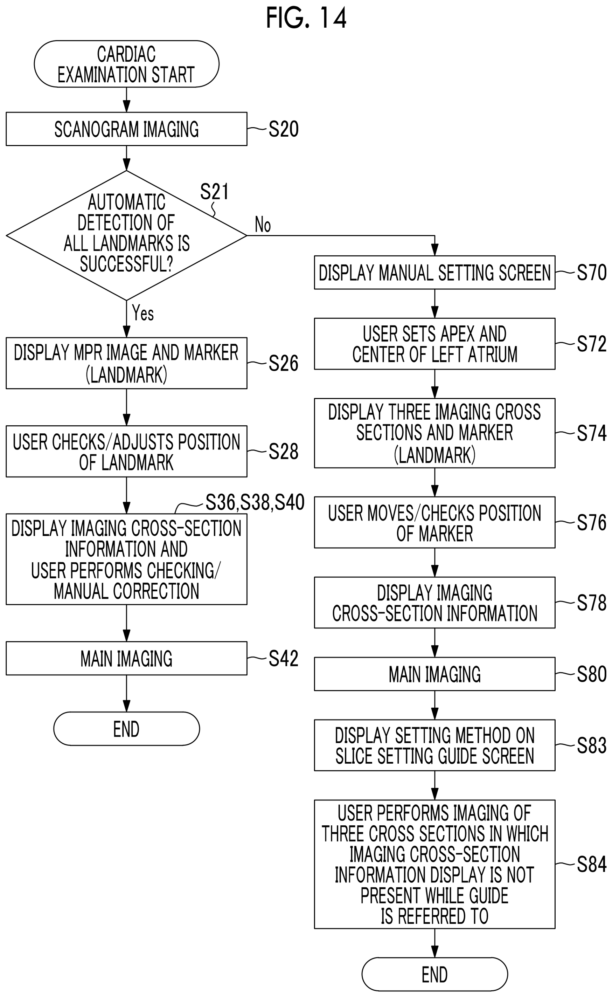

FIG. 14 is a flowchart showing an example of a procedure of a heart examination according to a modification example of the second embodiment. FIG. 14 shows a case where the imaging position applied to the main imaging is manually set and the main imaging is performed by an inexperienced radiologist. In such a case, step S83 and step S84 shown in FIG. 14 are executed instead of step S82 shown in FIG. 11.

In step S83, the slice setting guide screen for assisting in the manual setting of the slice is displayed on the display device 208 shown in FIG. 1. In step S84, the radiologist who is the user sets three cross sections that are not displayed on the manual setting screen 400A shown in FIG. 13 with reference to the slice setting guide screen, and performs the imaging of the three cross sections.

According to the modification example of the second embodiment, even the radiologist who is inexperienced in the manual setting of the imaging position can perform the manual setting of the preferable imaging position.

### Specific Example of Display Mode of Marker

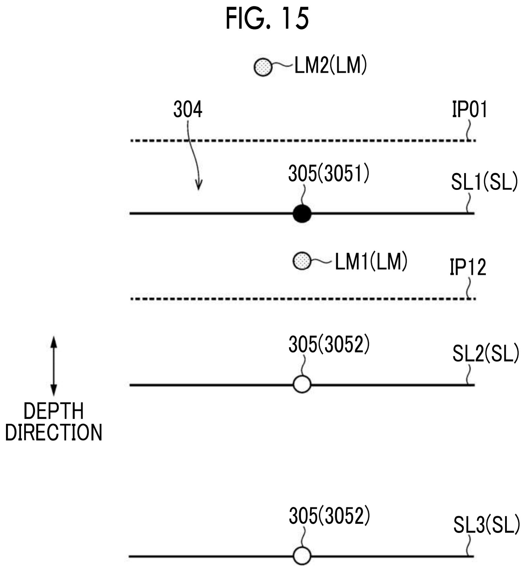

FIG. 15 is a schematic diagram showing an example of the display mode of the marker. The first slice SL1, the second slice SL2, and the third slice SL3 shown in the same drawing represent the positions in the depth direction with respect to the cross section for which the MPR image 304 is generated. An arrow line shown in FIG. 15 indicates the depth direction.

In a case in which the marker 305 representing the position of the landmark LM is displayed in a superimposed manner on the MPR image 304, a first display mode 3051 of the marker 305 is applied to the first slice SL1 closest to the landmark LM1.

For example, in a case in which the landmark LM1 is at a position between the first slice SL1 and the second slice SL2 and is located on the first slice SL1 side with respect to an intermediate position IP12 between the first slice SL1 and the second slice SL2, the first display mode 3051 is applied to the marker 305 displayed on the first slice SL1.

On the other hand, for the landmark LM1, a second display mode 3052 is applied to the marker 305 displayed on the second slice SL2 and the third slice SL3. In FIG. 15, a solid circle is shown as the first display mode 3051 of the marker 305, and a hollow circle is shown as the second display mode 3052.

In a case in which the position of the cross section for which the MPR image 304 is generated in the depth direction is separated from one end or the other end by a certain distance or more, as in the landmark LM2, the marker 305 is not displayed on any slice SL. The position of the landmark LM in the depth direction of the MPR image 304 is understood based on the display mode of the marker 305 displayed in a superimposed manner on the MPR image 304 shown in FIG. 15.

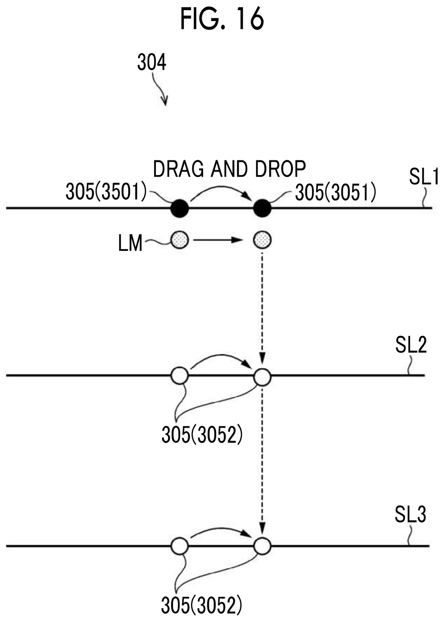

FIG. 16 is a schematic diagram in a case in which the marker is moved in the plane of the MPR image. FIG. 16 shows a case in which the first slice SL1 is displayed as the MPR image 304 and the marker 305 is moved in the plane of the first slice SL1.

In the first slice SL1 to which the first display mode 3051 is applied, for example, the marker 305 can be moved from a position to which the marker 305 is dragged to a position to which the marker 305 is dropped by applying drag and drop.

In a case in which the position of the landmark LM is corrected in accordance with the movement of the marker 305, the marker 305 is also moved in accordance with the corrected position of the landmark LM in the second slice SL2 and the third slice SL3 to which the second display mode 3052 is applied.

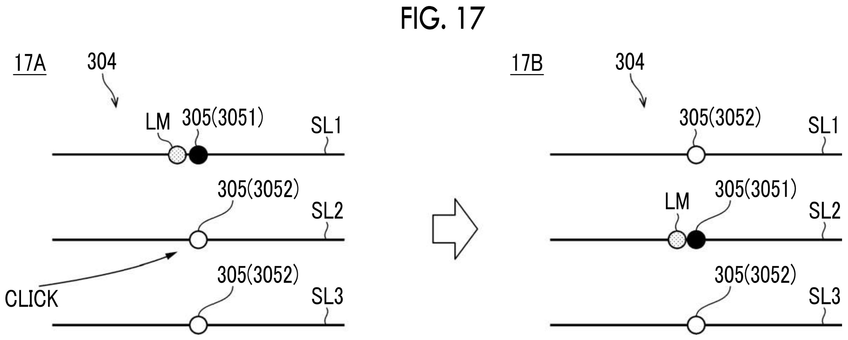

FIG. 17 is a schematic diagram in a case in which the marker is moved in the depth direction of the MPR image. A schematic diagram 17A in FIG. 17 shows a state before the landmark LM is moved in the depth direction of the MPR image 304, and a schematic diagram 17B in FIG. 17 shows a state after the landmark LM is moved in the depth direction of the MPR image 304. For convenience of illustration, the position of the landmark LM is shifted in the schematic diagram 17A and the schematic diagram 17B, but the position of the landmark LM matches the position of the marker 305.

In a case in which the second slice SL2, to which the second display mode 3052 is applied, is displayed in the marker 305, and the marker 305 is clicked in the second slice SL2, the landmark LM is moved from the position of the first slice SL1 to the position of the second slice SL2.

In the second slice SL2, the display mode of the marker 305 is changed from the second display mode 3052 to the first display mode 3051. On the other hand, in the first slice SL1, the display mode of the marker 305 is changed from the first display mode 3051 to the second display mode 3052.

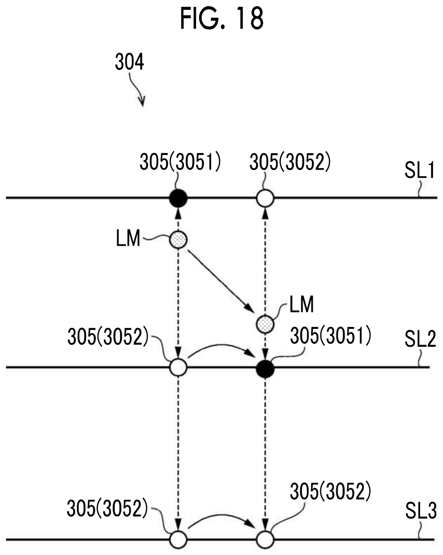

FIG. 18 is a schematic diagram showing an example of transition of the display mode of the marker. FIG. 18 schematically shows a change in the display mode of the marker 305 applied to the MPR image 304 of another cross section in a case in which the position of the marker 305 is moved in the plane of the MPR image 304 of any cross section.

Examples of any cross section include the vertical long-axis cross section shown in FIG. 3. In a case in which the vertical long-axis cross section shown in FIG. 3 is any cross section, examples of the other cross sections include a horizontal long-axis cross section, a short-axis cross section, a four-chamber cross section, a two-chamber cross section, and a three-chamber cross section.

In a case in which the marker 305 is moved in the plane of the MPR image 304 in any cross section, the position of the landmark LM is recalculated for the MPR image 304 in the other cross section based on the position of the landmark LM in the MPR image 304, and the recalculated position of the landmark LM is displayed.

In the MPR image 304 in the other cross section, the correction of the landmark LM may involve movement in the depth direction. In a case in which the correction of the landmark LM involves movement in the depth direction, the position of the slider 309 is changed in the other cross section, and the MPR image 304 is displayed for the cross section at the position in the depth direction corresponding to the position of the slider. The marker 305 displayed on the MPR image 304 is applied with the first display mode 3051.

As shown in FIG. 18, in a case in which the landmark LM at the position displayed on the first slice SL1 is moved to the position displayed on the second slice SL2, the display of the MPR image 304 is changed from the first slice SL1 to the second slice SL2, and the marker 305 in the second slice SL2 is changed from the second display mode 3052 to the first display mode 3051.

In a case in which the movement of the landmark LM involves movement in the depth direction, the display of the MPR image 304 in the other cross section may be performed by maintaining the position of the slider 309 and not changing the displayed slice SL, while moving the position of the marker 305 in the plane and changing the marker 305 from the first display mode 3051 to the second display mode 3052.

In any of the two types of displays of the MPR image 304 described above, the user can understand that the movement of the landmark LM in the depth direction of the MPR image 304 has occurred due to the correction of the landmark LM in the plane.

### Electric Configuration of Imaging Support Apparatus

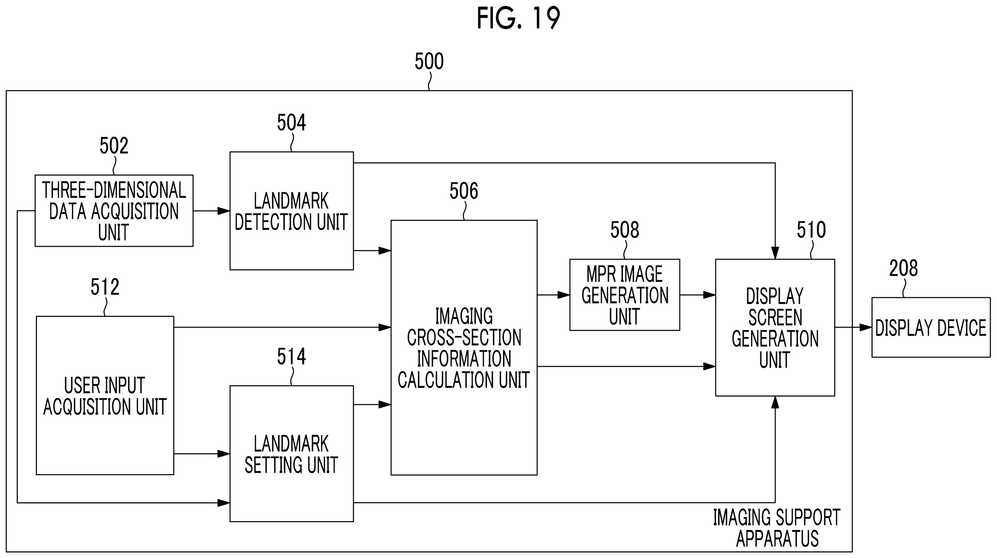

FIG. 19 is a functional block diagram of the imaging support apparatus. The control device 200 shown in FIG. 1 has a function as an imaging support apparatus 500 shown in FIG. 19.

The imaging support apparatus 500 comprises a three-dimensional data acquisition unit 502. The three-dimensional data acquisition unit 502 acquires the three-dimensional data obtained by performing the scanogram imaging in step S20 shown in FIG. 2.

The imaging support apparatus 500 comprises a landmark detection unit 504. The landmark detection unit 504 detects the specified landmark from the three-dimensional data in step S22. The landmark detection unit 504 may output a signal indicating that the landmark detection has failed in step S21 shown in FIG. 11.

The imaging support apparatus 500 comprises an imaging cross-section information calculation unit 506. The imaging cross-section information calculation unit 506 calculates the imaging cross-section information based on the landmark LM acquired by the landmark detection unit 504. Examples of the imaging cross-section information include information on reference six cross sections such as a vertical long-axis cross section shown in FIG. 3 and information on a cross section shown in FIG. 12.

The imaging support apparatus 500 comprises an MPR image generation unit 508. The MPR image generation unit 508 generates the MPR image 304 for each imaging position calculated using the imaging cross-section information calculation unit 506 in step S24.

The imaging support apparatus 500 comprises a display screen generation unit 510. The display screen generation unit 510 generates the automatic setting screen 300 shown in FIG. 3, the automatic setting screen 300A shown in FIG. 6, the manual setting screen 400 shown in FIG. 12, and the manual setting screen 400A shown in FIG. 13. The display screen generation unit 510 superimposes and displays a marker 305 representing the position of the landmark LM detected by the landmark detection unit 504 on the MPR image 304.

The display screen generation unit 510 transmits a display signal representing the automatic setting screen 300 or the like to the display device 208. The display device 208 displays the automatic setting screen 300 or the like.

The imaging support apparatus 500 comprises a user input acquisition unit 512. The user input acquisition unit 512 acquires a signal representing information input by the user operating various buttons provided on the automatic setting screen 300 or the like.

The imaging support apparatus 500 comprises a landmark setting unit 514. The landmark setting unit 514 sets the landmark LM in the three-dimensional data acquired by the three-dimensional data acquisition unit 502 based on a signal representing the position of the landmark LM manually input by the user. The imaging cross-section information calculation unit calculates the imaging cross-section information based on the manually set landmark LM. The display screen generation unit 510 displays the marker 305 representing the position of the manually set landmark LM in a superimposed manner on the MPR image 304.

### Regarding Computer and Processor Applied to Each Processing

In the present embodiment, each type of processing is executed by any computer. In addition, any computer may execute the processing by a processor, a program, or a combination thereof. Any computer may be a general-purpose computer, a computer for a specific application, a system such as a workstation, or other hardware elements capable of executing a program.

The processor may be configured by one or more hardware components, and the types of hardware are not limited. For example, the processor can be configured with a central processing unit (CPU), a micro processing unit (MPU), a programmable logic device such as a field programmable gate array (FPGA), a dedicated circuit for executing specific processing such as an application specific integrated circuit (ASIC), or hardware such as a graphic processing unit (GPU) or a neural processing unit (NPU). In addition, the processor has each unit or each means that executes various types of processes in the present embodiment. Additionally, the type of hardware may be a combination of different types of hardware. In a case in which the plurality of types of hardware components are configured to execute one or a plurality of processes of a certain processor, the plurality of types of hardware components may be present in devices physically separated from each other or may be present in the same device. In any embodiment, an order of each type of processing performed by the processor is not limited to the above order and may be appropriately changed. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and saved in a plurality of non-transitory computer-readable media present in apparatuses physically separated from each other. The program code or the code segment may represent any combination of a procedure, a function, a subprogram, a routine, a subroutine, a module, a software package, a class, an instruction, a data structure, or a program statement. The program code or the code segments may be connected to other code segments or hardware circuits by transmitting and receiving information, data, an argument, a parameter, or contents of a memory.

### Application Example to Program and Program Product

A program or a program product that causes a computer to realize some or all of various functions in the imaging support apparatus 500 and the method of operating the imaging support apparatus according to the embodiment can be stored in a computer readable medium that is an optical disk, a magnetic disk, a semiconductor memory, or another non-transitory information storage medium that is a tangible object, and the program or the program product can be provided through the information storage medium.

Instead of the above-described aspect in which the program is stored in the non-transitory computer readable medium that is a tangible object and applied, it is also possible to provide a program signal as a download service using an electric communication line.

Further, some or all of the functions in the imaging support apparatus 500 may be realized as cloud computing and may be provided as SaaS. It should be noted that SaaS is an abbreviation for Software as a Service.

The present disclosure is not limited to the above embodiments, and various modifications can be made within the scope that does not depart from the gist of the technical idea of the present disclosure. In addition, the first embodiment, the second embodiment, and the modification examples thereof can be combined as appropriate.

### Explanation of References

10: MRI apparatus
17A: schematic diagram
17B: schematic diagram
100: measurement unit
102: examinee
103: bed
104: static magnetic field generating magnet
106: gradient magnetic field coil
108: RF coil
110: receive coil
112: high-frequency magnetic field generator
114: gradient magnetic field power supply
116: receiver
118: sequencer
200: control device
202: processor
204: memory
206: input/output interface
208: display device
210: operation unit
300: automatic setting screen
300A: automatic setting screen
302: MPR image display region
304: MPR image
305: marker
306: window
307: slider bar
309: slider
310: first setting screen display switching button
311: second setting screen display switching button
314: landmark selection button
316: manual setting button
318: reset button
320: update image button
322: apply button
330: landmark position sample region
332: landmark position sample image
332A: landmark position sample image
332B: landmark position sample image
334: character information
334A: character information
400: manual setting screen
400A: manual setting screen
402A: first MPR image display region
402B: second MPR image display region
404AX: transverse cross-sectional image
404SA: short-axis cross-sectional image
404SAG: sagittal plane image
404VLA: vertical long-axis cross-sectional image
406: window
407: slider bar
409: slider
414: landmark selection button
420: update image button
422: apply button
424: next button
430: landmark sample region
432A: transverse cross-sectional sample position image
432B: sagittal plane sample position image
434: character information
500: imaging support apparatus
502: three-dimensional data acquisition unit
504: landmark detection unit
506: imaging cross-section information calculation unit
508: MPR image generation unit
510: display screen generation unit
512: user input acquisition unit
514: landmark setting unit
3051: first display mode
3052: second display mode
S10 to S84: each step of imaging support method

## Claims

1. An imaging support apparatus that supports imaging performed by a magnetic resonance imaging apparatus, the imaging support apparatus comprising:
a processor; and a memory,
wherein the processor is configured to:
acquire three-dimensional data including a heart of an examinee, the three-dimensional data being acquired in scanogram imaging performed using the magnetic resonance imaging apparatus before main imaging;
detect a landmark of the heart from the three-dimensional data;
calculate, based on a position of the landmark, a plurality of cross sections applied to imaging of the heart;
generate a plurality of cross-sectional images appearing in each of the plurality of cross sections from the three-dimensional data;
display the plurality of cross-sectional images;
display a first movement element representing a position of the landmark in a plane of the respective cross-sectional images; and
display a second movement element representing a position of the landmark in a depth direction of the respective cross-sectional images.

2. The imaging support apparatus according to claim 1,
wherein the processor is configured to:
receive, for any one cross-sectional image of the plurality of cross-sectional images, an operation of moving the first movement element; and
move, in response to the operation of moving the first movement element, the position of the landmark in the plane of the one cross-sectional image.

3. The imaging support apparatus according to claim 2,
wherein the processor is configured to:
move, in response to the movement of the position of the landmark in the plane of the one cross-sectional image, the position of the landmark in the plane of another cross-sectional image.

4. The imaging support apparatus according to claim 3,
wherein the processor is configured to:
display, for the other cross-sectional image, using the second movement element, a position to which the landmark is moved in the depth direction in a case where the position of the landmark in the depth direction is moved in response to the movement of the position of the landmark in the plane.

5. The imaging support apparatus according to claim 4,
wherein the processor is configured to:
change, for the other cross-sectional image, a display mode of the first movement element in a case where the position of the landmark in the depth direction is moved.

6. The imaging support apparatus according to any one of claims 1 to 5,
wherein the processor is configured to:
receive, for any one cross-sectional image of the plurality of cross-sectional images, an operation on the second movement element; and
move, in response to an operation of moving the position of the landmark in the depth direction represented by the second movement element, the position of the landmark in the depth direction of the one cross-sectional image.

7. The imaging support apparatus according to claim 6,
wherein the processor is configured to:
change, for any one cross-sectional image of the plurality of cross-sectional images, a display mode of the first movement element in a case where the position of the landmark in the depth direction is moved.

8. The imaging support apparatus according to any one of claims 1 to 7,
wherein the processor is configured to apply different display modes to the first movement element in accordance with the position of the landmark in the depth direction.

9. The imaging support apparatus according to any one of claims 1 to 8,
wherein the processor is configured to:
automatically detect the landmark of the heart from the three-dimensional data.

10. The imaging support apparatus according to any one of the preceding claims,
wherein the processor is configured to:
receive a manual setting of the position of the landmark for one or more predefined cross-sectional images.

11. A magnetic resonance imaging apparatus that generates an image of an examinee based on nuclear magnetic resonance, the magnetic resonance imaging apparatus comprising:
the imaging support apparatus according to any one of claims 1 to 10.

12. A method of operating an imaging support apparatus, the method comprising: causing a computer that functions as the imaging support apparatus that supports imaging performed by a magnetic resonance imaging apparatus to execute:
a step of acquiring three-dimensional data including a heart of an examinee, the three-dimensional data being acquired in scanogram imaging performed using the magnetic resonance imaging apparatus before main imaging;
a step of detecting a landmark of the heart from the three-dimensional data;
a step of calculating, based on a position of the landmark, a plurality of cross sections applied to imaging of the heart;
a step of generating a plurality of cross-sectional images appearing in each of the plurality of cross sections from the three-dimensional data;
a step of displaying the plurality of cross-sectional images;
a step of displaying a first movement element representing a position of the landmark in a plane of the respective cross-sectional images; and
a step of displaying a second movement element representing a position of the landmark in a depth direction of the respective cross-sectional images.

13. A non-transitory, computer readable recording medium which records thereon a program for causing a computer to execute the method of operating an imaging support apparatus according to claim 12.
